# EUROPEAN PATENT APPLICATION

(11) **EP 0 922 444 A1**
(43) Date of publication of application: **16.06.1999**
(21) Application number: 97121995.1
(22) Date of filing: 11.12.1997
(51) Int. Cl.: A61F 13/15, B32B 3/10

(54) **The use of a breathable absorbent article to provide a clean and dry topsheet**

(71) Applicant: THE PROCTER & GAMBLE COMPANY, Cincinnati, Ohio 45202 (US)
(72) Inventor: Bewick-Sonntag, Christopher Phillip, Dr., 65125 Pescara (IT); Di Cintio, Achille, 65126 Pescara (IT); Carlucci, Giovanni, 66100 Chieti (IT); Divo, Michael, 61381 Friedrichsdorf (DE); Cimini, Carmine, 65126 Pescara (IT)
(74) Representative: Kohol, Sonia

(57) **Abstract**

The present invention relates to absorbent articles such as sanitary napkins and the use of breathable backsheet constructions therein to provide improved topsheet performance. In particular, the present invention relates to a moisture vapour permeable, liquid impermeable backsheet and its use for the provision of a dry wearer facing surface, preferably a topsheet of an absorbent article.

## Description

### Field of the Invention

The present invention relates to absorbent articles and in particular sanitary napkins and the use of breathable backsheet constructions therein to provide improved topsheet performance.

### Background of the Invention

Absorbent articles such as sanitary napkins, diapers, incontinence products and perspiration pads are well known in the art. Typically these articles comprise a wearer facing surface and a garment facing surface. The wearer facing surface receives discharges from the body such as urine, faces, vaginal discharges and the like which are to be absorbed and stored by the article. In order for the article to absorb the discharges the wearer facing surface of the articles needs to be liquid permeable. This wearer facing surface is known as the topsheet.

Topsheets are also well known in the art and are typically selected nonwovens, wovens or apertured film materials. Wovens and nonwovens are desirable in so far as they provide a clothlike appearance to the topsheet and appear relatively soft and comfortable to the wearer. However, the problem with such topsheets is that they also have some capacity to absorb. The topsheet thus rapidly becomes saturated and feels wet to the wearer of the product. Furthermore, such woven or nonwoven topsheets also rapidly develop a used, unclean and unsanitary appearance. Attempts at remedying these problems however, by the use of surface treatments for example have not proven particularly satisfactory. The alternative proposed topsheets are apertured formed films which have funneled apertures which are designed to promote the passage of liquid discharge through the topsheet and into the absorbent core. These types of topsheet are preferred in that the problem of rewet is usually avoided. However a particular problem associated with these topsheets is that the topsheet feels very sticky to the wearer of the product and may in some circumstances adhere to the wearer's skin during use, which is highly uncomfortable. Moreover due to polymeric nature of the topsheets the topsheet may also become warmer during use further attributing to the uncomfortable impression experienced by the wearer during use.

Another problem common to the topsheets described above is related to the rate at which the topsheet is capable of allowing liquid to pass through it under normal usage conditions. The rate is typically dependent upon the total amount of open area of the apertures, and the individual shape and size of the apertures of the topsheet. Exceptionally large apertures increase the rate of liquid passage but pose problems related to the manufacture of such topsheets, particularly as far as the stability of the topsheet material itself is concerned. Additionally, large apertures may also promote a backflow of the absorbed liquid known as rewet. This is particularly undesirable as the wearer of the product experiences a wet feeling during the entire wearing time of the product and promotes unnecessary product replacement changes. Small apertures on the other hand, cannot provide the required liquid passage rate through the topsheet.

The problem of the rate of liquid passage through the topsheets is further exacerbated by liquids of high surface tension, high viscosity or liquids having a high solids content such as coagulated blood which may cause complete blockage of the apertures. In addition to actual rate of liquid discharge passage through the topsheet account must also be taken of the changes in the rate of liquid discharged from the user's body and of the variation in the viscosity of liquid discharged from person to person and over the time period of a woman's period. Hence, the topsheet must be able to cope with considerable fluctuations in the rate of liquid discharge from the wearer in order to prevent liquid collecting on the topsheet and causing the wearer to feel wet and uncomfortable.

Yet another problem particularly acute for topsheets of an apertured formed film type, is that due to the presence of integral apertures, skin irritation such as chaffing and skin soreness may be caused further increasing the discomfort experienced by the wearer.

Hence there exists a need to improve the currently utilised topsheet materials in order that they appear soft and comfortable to the wearer whilst not compromising on their ability to promote liquid passage without rewet and thereby maintain a dry surface.

It has now been surprisingly found that the problems associated with topsheets can be addressed by the incorporation and use of breathable backsheet constructions whether the absorbent article.

In particular it has been surprisingly found that the use of breathable backsheet constructions within absorbent articles provides a topsheet which appears clean to the wearer, due to the absence of liquid residues and does not tend to adhere to the skin of the wearer, in addition to being dry. Hence the wearer of the product feels clean and dry and has minimal awareness that the product is in use.

### Summary of the Invention

The present invention relates to the use of a breathable absorbent article in particular having a moisture vapour permeable, liquid impermeable backsheet. Said article comprises a wearer facing surface and a garment facing surface and said backsheet comprises said garment facing surface. Accordingly, the present invention relates to the use of said backsheet to provide a dry wearer facing surface. Preferably said wearer facing surface comprises the topsheet of said article.

### Detailed Description of the Invention

The present invention relates to absorbent disposable articles such as sanitary napkins, panty liners, incontinence products, perspiration pads and baby diapers. According to the present invention these products comprise a wearer facing surface, and a garment facing surface. Typically, such products comprise a liquid pervious topsheet providing the wearer facing surface, a backsheet providing the garment facing surface and an absorbent core intermediate said topsheet and said backsheet. The present invention relates to such articles which comprise a moisture vapour permeable, liquid impervious backsheet more commonly referred to as a breathable backsheet.

The absorbent articles can also comprise any of the components or features usual in the art, in particular side wrapping elements, side flap components, or wings as well as any sort of extensibility or elastication feature can be comprised in absorbent articles. For example a typical sanitary napkin or panty liner comprises an adhesive area on the garment facing surface of the backsheet providing the panty-fastening adhesive which is covered by a release paper, wrapper or the like prior to the use of the article.

The absorbent article for absorbing liquid is described below by reference to a sanitary napkin or panty liner. However products such as adult or baby diapers, incontinence products or perspiration pads can similarly benefit from the present invention.

### Backsheet

The absorbent article according to the present invention comprises as an essential feature a breathable backsheet. The backsheet primarily prevents the extrudes absorbed and contained in the absorbent structure from wetting articles that contact the absorbent product such as underpants, pants, pyjamas and undergarments thereby acting as a barrier to fluid transport. The backsheet typically extends across the whole of the absorbent structure and can extend into and form part of, or all of the sideflaps, side wrapping elements or wings. However, in addition to acting as a liquid barrier, the breathable backsheet of the present invention permits the transfer of at least moisture vapour, preferably both vapour and air through it and thus allows the circulation of gases into and out of the backsheet. It has now been further surprisingly identified that the utilisation of a breathable backsheet also delivers desirable benefits to the topsheet of the article. In particular, the use of a breathable backsheet provides a clean and dry facing surface, preferably the topsheet, such that the surface feels dry to the touch and the skin of the wearer of the article does not feel wet or moist and such that the wearer experiences minimal discomfort during wearer.

According to the present invention suitable breathable backsheets suitable to provide the benefits as described herein above may be any breathable backsheet known in the art comprising at least one moisture vapour permeable layer. Suitable moisture vapour permeable layers include 2 dimensional, planar micro and macro-porous films; macroscopically expanded films; formed apertured films;monolithic films and nonwoven layers. According to the present invention the apertures in said layer may be of any configuration, but are preferably spherical or oblong and may also be of varying dimensions. The apertures preferably are evenly distributed across the entire surface of the layer, however layers having only certain regions of the surface having apertures are also envisioned.

Suitable 2 dimensional porous planar layers of the backsheet may be made of any material known in the art, but are preferably manufactured from commonly available polymeric materials. Suitable materials are for example Goretex (TM) or Sympatex (TM) type materials well known in the art for their application in so-called breathable clothing. Other suitable materials include XMP-1001 of Minnesota Mining and Manufacturing Company, St. Paul, Minnesota, USA and Exxaire XBF-101W, supplied by the Exxon Chemical Company. As used herein the term 2 dimensional planar layer refers to layers having a depth of less than 1 mm, preferably less than 0.5mm, wherein the apertures have an average uniform diameter along their length and which do not protrude out of the plane of the layer. The apertured materials for use as a backsheet in the present invention may be produced using any of the methods known in the art such as described in EPO 293 482 and the references therein. In addition the dimensions of the apertures produced by this method may be increased by applying a force across the plane of the backsheet layer (i.e. stretching the layer).

Suitable apertured formed films include films which have discrete apertures which extend beyond the horizontal plane of the garment facing surface of the layer towards the core thereby forming protuberances. The protuberances have an orifice located at its terminating end. Preferably said protuberances are of a funnel shape, similar to those described in US 3, 929,135. The apertures located within the plane and the orifices located at the terminating end of protuberance themselves maybe circular or non circular provided the cross sectional dimension or area of the orifice at the termination of the protuberance is smaller than the cross sectional dimension or area of the aperture located within the garment facing surface of the layer. Preferably said apertured performed films are uni directional such that they have at least substantially, if not complete one directional fluid transport towards the core.

Suitable macroscopically expanded films for use herein include films as described in for example in US 4 637 819 and US 4 591 523.

Suitable monolithic films include Hytrel™, available from DuPont Corporation, USA, and other such materials as described in Index 93 Congress, Session 7A "Adding value to Nonwovens", J-C. Cardinal and Y. Trouilhet, DuPont de Nemours international S.A, Switzerland such as Pebax™, available from Elf Atochem (France) and Estane ™ available from BF Goodrich (Belgium).

Preferred breathable backsheets for use herein are those having a high moisture vapour exchange, most preferably both a high moisture vapour and high air exchange. Particularly preferred backsheets for the present invention comprise at least two layers comprising at least one layer selected from the above, such as a microporous layer or an apertured formed film and an additional layer which may also be selected from the above listed backsheets. The most preferred breathable backsheet component comprises a microporous film and an apertured formed film; or a microporous film and a hydrophobic fibrous layer; or an apertured formed film and a hydrophobic fibrous layer. Preferably, the hydrophobic fibrous layers are hydrophobic non woven.

### The topsheet

According to the present invention it has been found that any of the typically utilised topsheets in absorbent articles can obtain a dryness benefit from the use of the breathable backsheet.

The topsheet is compliant, soft feeling, and non-irritating to the wearer's skin. The topsheet also can have elastic characteristics allowing it to be stretched in one or two directions in portions of the topsheet or throughout its extension. Further, the topsheet is fluid pervious permitting fluids (e.g., menses and/or urine) to readily penetrate through its thickness. A suitable topsheet can be manufactured from a wide range of materials such as woven and nonwoven materials; polymeric materials such as apertured formed thermoplastic films, apertured plastic films, and hydroformed thermoplastic films; and thermoplastic scrims and combinations thereof. Suitable woven and non woven materials can be comprised of natural fibers (e.g., wood or cotton fibers), synthetic fibers (e.g., polymeric fibers such as polyester, polypropylene, or polyethylene fibers) or from a combination of natural and synthetic fibers or bi-/multi-component fibers and are preferably hydrophobic.

Preferred topsheets for use in the present invention are selected from high loft nonwoven topsheets and apertured formed film topsheets. Apertured formed films are especially preferred for the topsheets because they are pervious to body exudates and yet non absorbent and have a reduced tendency to allow fluids to pass back through and rewet the wearer's skin. Thus, the surface of the formed film that is in contact with the body remains dry, thereby reducing body soiling and creating a more comfortable feel for the wearer. Suitable formed films are described in U.S. Patent 3,929,135; U.S. Patent 4,324,246; U.S. Patent 4,342,314; U.S. Patent 4,463,045; and U.S. Patent 5,006,394. Particularly preferred micro apertured formed film topsheets are disclosed in U.S. patent 4,609,518 and U.S. patent 4,629,643. A preferred topsheet for the present invention comprises the formed film described in one or more of the above patents and marketed on sanitary napkins by The Procter & Gamble Company of Cincinnati, Ohio as "DRI-WEAVE".

Topsheets having not a homogeneous distribution of liquid passage ways but only a portion of the topsheet comprising liquid passage ways are also contemplated by the present invention. Typically such topsheets would have the liquid passage ways oriented such that they result in a centrally permeable and peripherally impermeable topsheet for liquids.

The wearer facing surface of the formed film topsheet can be hydrophilic so as to help liquid to transfer though the topsheet faster than if the body surface was not hydrophilic. In a preferred embodiment, surfactant is incorporated into the polymeric materials of the formed film topsheet such as is described in PCT-publication WO 93/09741. Alternatively, the body surface of the topsheet can be made hydrophilic by treating it with a surfactant such as is described in U.S. 4,950,254.

Another alternative are so called hybrid topsheets which incorporate fibrous and film like structures. Particularly useful embodiments of such hybrid topsheets are disclosed in PCT publications WO 93/09744; WO 93/11725 or WO 93/11726 and US 4 780 352.

When referring to the topsheet a multi layer structure or a mono layer structure is contemplated. The hybrid topsheet mentioned above is such a multi layer design but other multi layer topsheets such as primary and secondary topsheet designs are also considered.

The topsheet typically extends across the whole of the absorbent structure and outside the area coextensive with the absorbent structure. The topsheet can extend and form part or all of the preferred side flaps, side wrapping elements or wings.

### Absorbent core

According to the present invention the absorbent cores suitable for use in herein may be selected from any of the absorbent cores or core system known in the art. As used herein the term absorbent core refers to any material or multiple material layers whose primary function is to absorb, store and distribute fluid.

According to the present invention, the absorbent core can include the following components: (a) an optional primary fluid distribution layer preferably together with a secondary optional fluid distribution layer; (b) a fluid storage layer; (c) an optional fibrous ("dusting") layer underlying the storage layer; and (d) other optional components.

### a Primary/Secondary Fluid Distribution Layer

One optional component of the absorbent core according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials usually referred to as "hydrogel", "superabsorbent", hydrocolloid" materials in combination with suitable carriers.

The absorbent gelling materials are capable of absorbing large quantities of aqueous body fluids, and are further capable of retaining such absorbed fluids under moderate pressures. The absorbent gelling materials can be dispersed homogeneously or non-homogeneously in a suitable carrier. The suitable carriers, provided they are absorbent as such, can also be used alone.

Suitable absorbent gelling materials for use herein will most often comprise a substantially water-insoluble, slightly cross-linked, partially neutralised, polymeric gelling material. This material forms a hydrogel upon contact with water Such polymer materials can be prepared from polymerizable, unsaturated, acid-containing monomers which are well known in the art.

Suitable carriers include materials which are conventionally utilised in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

Another component which can be included in the absorbent core according to the invention and preferably is provided close to or as part off the primary or secondary fluid distribution layer are odor control agents.

A preferred sanitary napkin or panty liner made according to the present invention has a pair of side wrapping elements or "undergarment covering components". These elements or components provide coverage of the wearer's panties to reduce side soiling (i.e., staining of the edges of the panty crotch) and are typically smaller than conventional flaps or wings.

The function of the side wrapping elements, whether integral with the article or joined to the article after being formed separately, is further improved by rendering them extensible in one or both directions parallel to the longitudinal axis and/or lateral axis. The extensibility can be provided across all or only part of the side wrapping elements and can be achieved by pleating or ring-rolling those parts which are to be rendered extensible.

According to the present invention the topsheet, backsheet and absorbent core components are joined together to provide the absorbent article. Typically, at least two, preferably all of the components of the article are joined to form the article. Each of said components of the absorbent article comprise at least one layer and have a wearer facing surface and a garment facing surface. Typically, adjacent garment facing surfaces form a common interface with the wearer facing surface of an adjacent component or layer. The elements or layers are joined together across this common interface. In this manner, the topsheet is joined to the absorbent core, and the core is joined to the backsheet. In addition, the topsheet may be directly or indirectly by joined to the backsheet at the periphery of the absorbent article. Furthermore, particularly in sanitary napkin, panty liner and incontinence product applications, the garment facing surface of the backsheet also provides the surface to which the absorbent article is releasably joined to the garment of the user of the product. Prior to use, this surface is typically provided with a protective cover. Any means known in the art to join the components of the absorbent article and provide the garment fastening. May be utilised such as utilising a continuous layer of adhesive, a patterned layer of adhesive, such as spirals, or spots, or using heat bonds, pressure bonds, mechanical bonds and the like.

## Claims

1. The use of a moisture vapour permeable, liquid impervious backsheet in an absorbent article, said article further comprising a wearer facing surface and a garment facing, said garment facing surface being provided by said backsheet in order to provide a dry wearer facing surface.

2. The use according to claim 1, wherein said absorbent article further comprises a topsheet and an absorbent core, said core being located in between said topsheet and said backsheet and wherein said topsheet provides said wearer facing surface.

3. The use according to claim 1 of said backsheet, wherein said backsheet comprises at least one layer selected from apertured polymeric formed films, 2-dimensional planar apertured films or nonwovens.

4. The use according to claim 3, wherein said backsheet comprises at least two layers, and wherein both of said layers are independently selected from apertured formed polymeric films and 2-dimensional planar apertured films.

5. The use according to claim 3, wherein said backsheet comprises at least two layers, a first layer comprising an apertured layer and a second layer comprising a fibrous layer.

6. The use according to claim 2, wherein said topsheet is a non absorbent topsheet, preferably a hydrophobic nonwoven or an apertured polymeric film.

7. The use according to claim 2, wherein said topsheet is an apertured formed polymeric film.

8. The use according to any one of the preceding claims, wherein said absorbent article is a sanitary napkin or a panty liner.
